# EUROPEAN PATENT SPECIFICATION

(11) **EP 3 422 948 B1**
(45) Date of publication and mention of the grant of the patent: **09.10.2024**
(21) Application number: 17707107.3
(22) Date of filing: 21.02.2017
(51) Int. Cl.: A61B 5/20, A61B 5/00

(54) **URINE WEIGHING APPARATUS**
URINWÄGEVORRICHTUNG
APPAREIL DE PESAGE D'URINE

(30) Priority: 29.02.2016 GB 201603449
(43) Date of publication of application: 09.01.2019
(73) Proprietor: University of Leicester, Leicester Leicestershire LE1 7RH (GB)
(72) Inventor: HOLT, John, Leicester Leicestershire LE1 7RH (GB); BUSTIN, Gareth, Leicester Leicestershire LE1 7RH (GB); SIMS, Mark, Leicester Leicestershire LE1 7RH (GB); COATS, Tim, Leicester Leicestershire LE1 7RH (GB)
(74) Representative: Hutter, Anton
(86) International application number: PCT/GB2017/050436
(87) International publication number: WO 2017/149272

(56) References cited:
- EP-A1- 2 730 215
- EP-A1- 2 799 096
- WO-A1-2009/107012
- WO-A2-01/82772
- US-A- 4 712 567
- US-A1- 2006 253 064

## Description

The present invention relates to urine weighing apparatuses, and particularly, although not exclusively, to gravimetric apparatuses for weighing urine and thereby measuring urine output from a subject, such as a catheterised patient. The invention also extends to uses of the apparatus, and to methods and systems of measuring the urine output from a patient.

Urine output corresponds to the total volume of urine output as well as the rate of urine output. According to a 2013 Nature paper [1], "...monitoring of urine output could thus serve as a continuous early warning system for acute kidney injury, akin to the caged canary in the coal mine"*.* However, urinary output of a patient, particularly in a hospitalised high dependency setting, is a critical parameter that, unlike most vital signs, is not electronically monitored [2] and recorded. Current systems used for measuring urine output rely on nursing staff making routine measurements by reading a scale on the catheter collection bag (vessel) and manually recording the figure on the patient's written "observations chart". Data from which can be entered into an electronic patient record where available. This manual task takes considerable nursing resources, is not always accurate depending on viewing angle, and is only conducted intermittently with changes in fluid output currently detected on typically 0.5 to 1 hour timescales for the most critically ill, but typically 4 to 6 hour intervals for other patients.

Thus, the current manual/visual system used for the measurement of urine output in a clinical setting has many disadvantages, including irregular times between measurements, which make it difficult for clinicians to be alerted to and rapidly identify changes in output rate. Also, matching a fluid level to a scale by eye leads to human error, especially if the bag is not level or if the bag is low down, this is particularly true when viewing the bag at an angle from a standing or sitting position or if the bag is in a dark environment. In addition, individual variation in measurement methods between personnel, for example different viewing angles, can mean that records sometimes make a 'step change' between nurse shifts. Furthermore, the sensitivity of the current visual system is limited, which makes it difficult to measure small changes in urine output. Transcription errors may often occur when manually entering data into either paper or electronic records and the integrity of data, reported in literature, has been of concern in many situations. Also, measurement is time intensive: Each conventional measurement takes one or two minutes and such measurements are completed as part of routine observations every 4 or 6 hours depending on the status of the patient. When multiplied by the number of catheterised patients this becomes a significant investment in staff resources. For example, in England during 2013, 15.1 million people were admitted to an NHS hospital and of these it is expected that between 15 to 25% would have an indwelling catheter [7] which amounts to over 10,000 patients per day needing routine measurement of their urine output.

Several devices have been created in an attempt to automate the monitoring and recording of urine output data from a catheterised patient. However, there is not an alternative solution currently in clinical use in the UK NHS. Several devices to measure urine flow in the past have been developed but with no success to date ([2]-[7]). Current proposed alternatives to the current visual/manual method of measuring urine output utilise a direct measuring system with a sensor that measures actual flow rates or specialist modified urine collection bags with an optical, capacitive or magnetic transducer. Such systems require complex and expensive technical interfaces with the voided urine. They also raise concerns with respect to sterility/cross contamination because they require regular cleaning, maintenance and such designs contain greater dead volumes. For example, WO 2008/059483 A2 discloses an optical sensor which requires a modified collection bag or an in-line fluid sensor module attached to the top of the collection bag and a wired remote display unit. A disadvantage of this sensor is that it interrupts the flow of urine and/or compromises the sterility of the catheter. Other devices have been described which measure urine output volume and flowrate based on urine mass. However, such apparatuses are designed for non-continuous use with non-catheterised patients, for example, in the assessment of lower urinary tract symptoms, such as those caused by prostate disorders. Also, such systems tend to be designed with an open urine inlet system akin to a container for use with a commode-type apparatus or similar. Known urine weighing devices are described in US 2006/0253064 A1, WO 2009/107012 A1, WO 01/82772 A2, US 4,712,567 A, and EP 2 799 096 A1.

There is therefore a need for an improved apparatus that automatically and continuously measures urine output.

Thus, according to a first aspect of the invention, there is provided a urine weighing apparatus according to claim 1.

Preferably, the apparatus according to the first aspect is configured to measure the weight of the vessel, and thereby calculate, in real-time, the volume of urine therein. Advantageously, the apparatus provides continuous, real-time measurement of urine output with existing (i.e. non-modified) urine collection bags. Thus, it is far cheaper to implement than alternative devices that only work in conjunction with additional specialised collection bags, catheters or other equipment.

In addition, the apparatus according to the disclosure can be used without compromising the sterility of the catheter, and thus minimises the possibility of infection. Furthermore, the apparatus can be used in addition to the conventional visual/manual approach. Further advantages of the invention include that the apparatus is reusable; it can be cleaned without having to disconnect the catheter (i.e. is easy to clean); and it can operate stand-alone. The invention may also be used to reduce the amount of time required to manually measure urine output on the scale of a urine collection bag, through the use of a 'display on device' feature or wireless transmission of data to a separate station, such as a nurse station display and allows patient trends to be monitored and deterioration or improvement to be identified quicker.

The apparatus is configured to measure urine output from a subject by calculating the amount of voided urine volume and flowrate using the change in weight of the urine collection vessel over time. Measurement of the weight of the urine collection vessel is achieved using the transducer. Any type of transducer that is capable of measuring the weight of the urine collection vessel and converting the change in weight into an electrical signal may be used in the apparatus of the first aspect. Preferably, the transducer is a load cell. The load cell may be an S-type load cell, a piezoelectric load cell, a shear beam load cell, a double-ended shear beam load cell or a rope clamp load cell. Preferably, the load cell is an S-type load cell or a piezoelectric load cell. Most preferably, the load cell is an S-type load cell with a minimum of two strain gauges configured to measure both positive and negative changes in force.

Current gravimetric devices used to measure urine output are not suitable for continuous use with catheterised patients in a clinical or care setting as they comprise static (*in situ*)*,* open containers which would be unacceptable from a hygiene perspective, and they require a flat stable base in order to record accurate and consistent measurements. Gravimetric devices measure the volume of voided urine using a weight transducer to measure the weight of urine collected in a urine collection vessel. Using this information, which can be obtained using a known gravimetric device, and the specific gravity of urine, the skilled person is able to accurately calculate the volume of urine produced. However, the flow rate of voided urine can only be measured at various time points (not continuously) with a time resolution of the order a few minutes. Also, known gravimetric devices would be unsuitable for measuring urine output as they are not portable and have no means of correcting for unwanted noise in the readings which may arise due to movement of the apparatus.

Unlike known gravimetric devices, the apparatus according to the first aspect enables continuous and portable measurement of voided urine volume over time. Preferably, the apparatus is configured to calculate the voided urine volume at a plurality of time points, and then calculate a rate of change of urine volume over the plurality of time points.

Preferably, the transducer is configured to create an electrical signal with a magnitude indirectly or directly proportional to the force being measured. The force being measured is the force applied to the support means by the voided urine in the urine collection vessel. The electrical signal measured may be electrical resistance, electrical current, electrical voltage or electrical charge. Each of these electrical signals is highly sensitive to changes in force being measured. Thus, they enable the invention to consistently provide highly accurate and precise readings. In embodiments in which the electrical signal is resistance, the magnitude of the resistance is indirectly or directly proportional to the force being measured. In embodiments in which the electrical signal is current, the magnitude of the current is indirectly or directly proportional to the force being measured. In embodiments in which the electrical signal is voltage, the magnitude of the voltage is indirectly or directly proportional to the force being measured. In embodiments in which the electrical signal is charge, the quantity of the charge is indirectly or directly proportional to the force being measured. Preferably, the electrical signal is voltage.

The force measured by the apparatus may be the weight of voided urine collected in the urine collection vessel. The apparatus may be capable of detecting a 0 - 100 N, 0 - 75 N or 0 - 50 N change of force. Preferably, the change of force is 0 - 50 N. The change of force may be less than 100 N, less than 90 N, less than 80 N, less than 70 N, less than 60 N, less than 50 N, less than 40 N, less than 30 N, less than 20 N or less than 10 N. The apparatus may be capable of detecting a change in voided urine volume of less than 1 ml, 0.75 ml, 0.5 ml, 0.3 ml or 0.1 ml. Preferably, the apparatus is capable of detecting a change in voided urine volume of less than 0.1 ml.

The support means is configured to support or elevate the urine collection vessel off of a surface, and preferably in-line with the load cell, or a means that may be used to fasten the load cell in-line with the urine collection vessel. Thus, the support means is capable of counteracting the force of gravity on the urine collection vessel. Preferably, the load cell is elevated or fastened in-line with the urine collection vessel so that substantially all of the force generated by the urine collection vessel is applied to the support means. Therefore, the load cell is operably linked to the urine collection vessel via the support means. The support means may be a hook, a latched hook (a hook with a moveable latch), a clip or cradle which supports the collection vessel. Preferably, the support means is a latched hook.

The urine collection vessel may be a urine collection bag, which is preferably sterile. The urine collection vessel may be a standard urine collection bag or an "hourly" urine collection bag. The volume of the standard urine collection bag is typically 1500 ml. The standard urine collection bag has a scale printed on the outside, such that when it is held up, and level, the volume contained is read from the value at the top of the scale. 'Hourly' urine collection bags have an additional rigid plastic chamber with a finer graduation printed on the side which, when the bag is held level, gives a more accurate measurement by matching the top of the fluid level with the scale, only if the rigid plastic chamber contains urine / fluid below its maximum value.

The apparatus may comprise an amplifier configured to amplify the electronic analogue signal generated by the transducer.

The apparatus may comprise a display for displaying the measured data referred to as 'display on device'. The display enables manual measurement with minimised possibility of human error. The display may be present on the apparatus in the absence of the apparatus being connected to a working computer. Therefore, the display is useful if a computer or wireless interface is unavailable. Preferably, the display is touch sensitive. The display may be a resistive touch display or ideally, a capacitive touch display type (which does not require actuator force), such that button areas on the display may be used by the clinical operator.

The apparatus may comprise both audible and/or visible (LED or display indicators) to indicate a particular status of the device, an error or a defined alarm situation or possible failure mode, for example low battery.

The apparatus may comprise a wireless module for transmission of data to a separate (i.e. spaced apart) electronic device, computer, server (web page), laptop, tablet or smart phone. The wireless module may be a wifi module, Bluetooth module or other radio data telemetry protocol.

The apparatus may comprise a power supply. The power supply may be a battery, or a battery and a charge pump, an alternative power management circuit, or a mains power adaptor. The power supply may use inductive charging, such that the battery can be recharged by a non-contact electromagnetic charging station. This enables a fully sealed enclosure (no power connector) facilitating ease and effective cleaning.

Preferably, the apparatus is configured to process the data to remove unwanted changes and data artefacts (i.e. noise) due to shock inputs, clinical interventions and/or movement of the apparatus and/or subject. Data processing may therefore be described as filtering or cleaning the data. Preferably, the processor is programmed with an algorithm which manages the fluid volume data, including interventions, such as bag emptying operations and urine sample collections, i.e. interventions that abruptly change the vessel's weight. In use, a clinical user will confirm intervention operations with a "Tare" function button on the apparatus. Hence, the apparatus preferably comprises a "Tare function" to allow correction for the collection vessel weight on start of measurement and subsequent interventions that would affect the urine monitoring. The Tare function may be a simple reset and scale zero function. Preferably, however, the Tare function is part of the "fluid monitoring" algorithm to track total urine volume; for example, when the collection vessel is emptied or a urine sample taken for other / pathology analysis. The processor may comprise a microprocessor.

In another embodiment, the algorithm may be present on a separate electronic device, such as a computer, a server (web page), a laptop, a tablet or a smart phone. The separate electronic device may comprise a central monitoring system or system(s) to generate appropriate alerts on either or the apparatus or separate electronic device.

Preferably, the apparatus is configured to produce an alert signal if the weight over a pre-determined time period exceeds or is lower than a set-point. The alert signal may comprise a display, such as a flashing light, or an alarm sound or electronic notification to a separate electronic device, computer, server (web page), laptop, tablet or smart phone. The electronic notification may be initiated by the apparatus itself, or via a central monitoring system, computer or laptop or directly on the separate electronic device. If the alert signal notifies a "full vessel" status, the algorithm, in conjunction with the 'Tare' function, will recognise a replaced or emptied bag and append subsequent data to that already collected.

According to a second aspect, there is provided use of the apparatus according to the first aspect, for weighing urine.

According to a third aspect of the invention, there is provided a method of measuring the urine output from a subject, the method comprising attaching the apparatus according to the first aspect to the subject, and measuring the urine output therefrom.

Preferably, the apparatus is attached to the subject by a catheter, or the like.

Preferably, the method comprises processing the data to remove unwanted changes and data artefacts due to shock inputs and movement.

According to a fourth aspect, there is provided a urine detection system comprising the apparatus according to the first aspect, and a urine collection vessel.

Preferably, the system comprises delivery means for delivering urine from a subject to the urine collection vessel, more preferably a catheter.

According to a fifth aspect, there is provided a urine detection system comprising the apparatus according to the first aspect, and at least one device for receiving data transmitted by the apparatus, which corresponds to the weight of the urine output.

The apparatus preferably comprises a wireless module for transmission of the data to the electronic device. The wireless module may be a wifi module, a Bluetooth module or a radio data telemetry protocol system approved for use in a medical environment. The at least one device for receiving the transmitted data is a separate (i.e. spaced apart) electronic device, for example, a computer, a laptop, a tablet or a smart phone. The apparatus and/or device may comprise an algorithm that processes the data and removes unwanted changes and data artefacts (i.e. noise) due to shock inputs and movement. The apparatus and/or device may comprise a central monitoring system or system(s) to generate appropriate alerts.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying diagrammatic drawings, in which:-
**Figure 1** shows a forward view of one embodiment (i.e. the MictuRATE MKII) of an apparatus for continuously measuring urine output according to the invention;
**Figure 2** is a block diagram of one embodiment of the apparatus used in the MKII shown in Figure 1;
**Figure 3** is a forward view showing the location of the apparatus in use by a catheterised patient on a hospital bed;
**Figure 4** is a schematic diagram showing the main components of a urine detection system in which the apparatus shown in Figure 1 & 2 can be used. However, Figure 4 shows a second embodiment of the apparatus incorporated in the system;
**Figure 5** shows a plan view of a tablet computer (used for testing) that may be wirelessly connected to the apparatus shown in Figure 1 and used in the system shown in Figure 4;
**Figure 6** shows examples of clinical raw and processed (with a processing algorithm) data generated using an embodiment of the invention (MictuRATE MK II). The upper panel shows raw data with intermittent noise events. In the lower panel the noise events have been removed; and

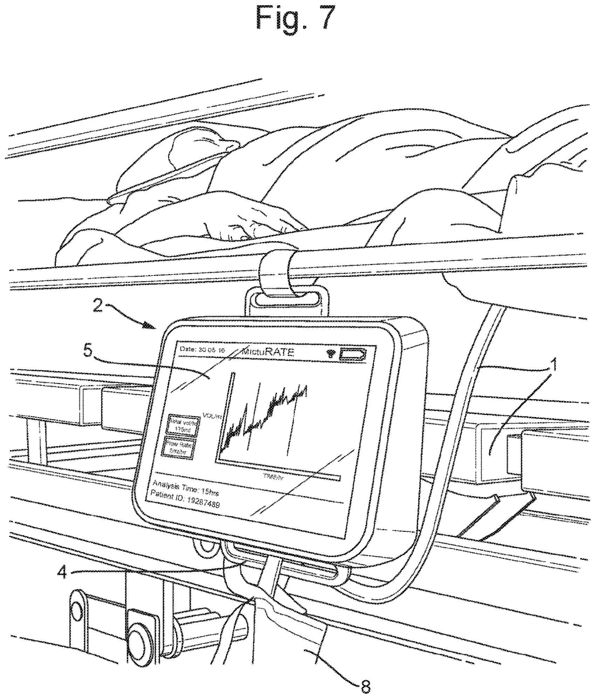

**Figure 7** shows a virtual embodiment of a MictuRATE apparatus for continuously measuring urine output according to the invention. Importantly, it shows how such a monitoring device may be used within a clinical environment.

### Examples

### Example 1 - Gravimetric urine output measuring apparatus

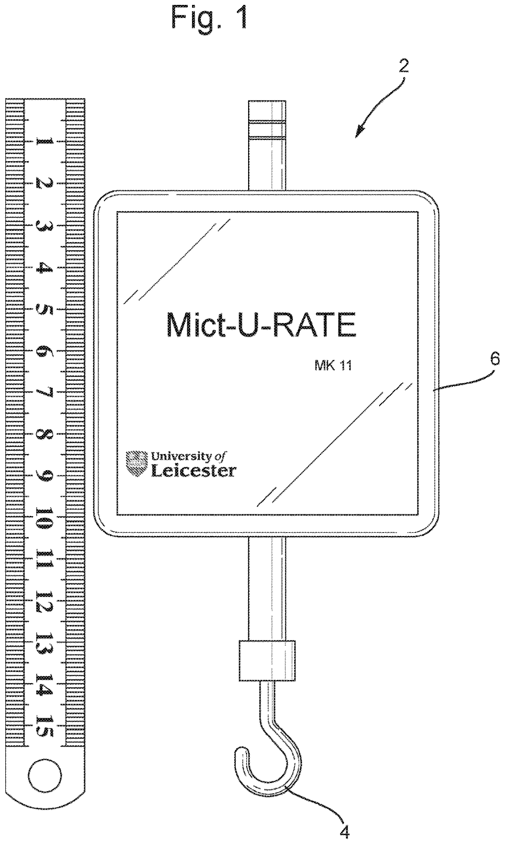

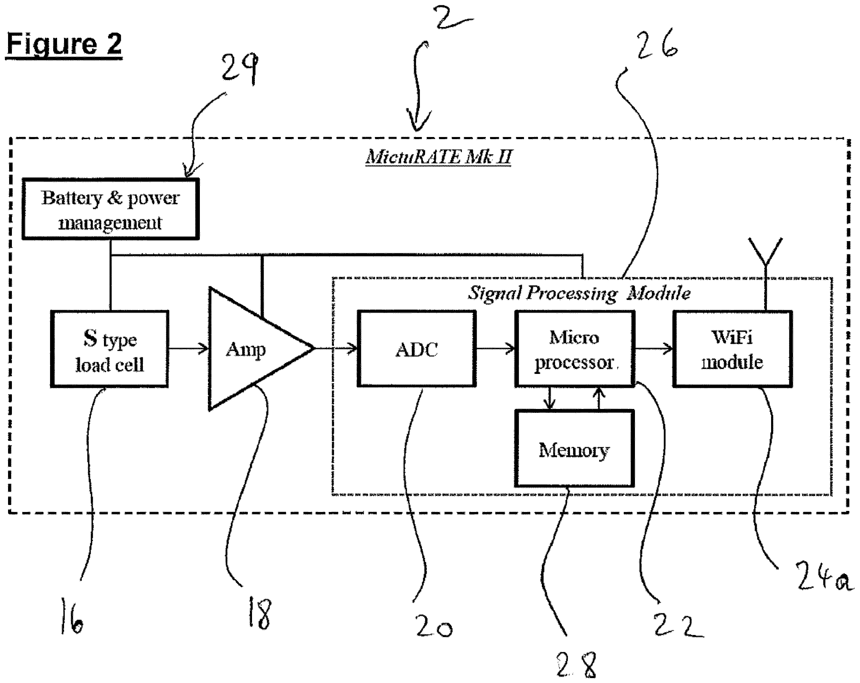

Referring to Figures 1 and 2, there is shown an embodiment of the gravimetric apparatus 2 according to the invention, which may, for example, be used to measure urine output from a catheterised patient. The gravimetric apparatus 2 comprises a miniature S-type load cell 16, which is operably linked to a hook 4 that is used to bear the weight of a urine collection bag 8. The hook 4 is positioned such that, when the apparatus 2 is in use, it is in-line with the urine collection bag 8 (i.e. the apparatus 2 is configured such that it has a vertical uniaxial arrangement with respect to the urine collection bag 8). Thus, strain/weight applied to the hook 4 by the urine collection bag 8 is converted into an analogue electrical signal by the S-type load cell 16.

Referring to Figure 2, there is shown a block diagram showing the inter-relationship between various components of one embodiment of the gravimetric apparatus 2 (referred to as "MictuRATE Mk II"). As can be seen, the S-type load cell 16 converts a change in force (i.e. strain), applied to the hook 4 by the urine bag 8, into an analogue electrical signal. The (analogue) electrical signal is typically collected over an interval of 1000ms at 1 MS/s at a resolution of 12 bits. The electrical signal is inversely proportional to the force being measured. The electrical signal is amplified by an amplifier 18 and then fed into an analogue-to-digital converter (ADC) 20, which is disposed within a signal-processing module 26. A microprocessor 22 converts the digital output data from the ADC 20 into comma separated values (CSV) and then transmits the converted data to a wifi module 24a. The microprocessor 22 exchanges data with a memory storage device 28. The wifi module 24a, the microprocessor 22 and the memory storage device 28 are also disposed within the signal-processing module 26. The wifi module 24a enables wireless telemetry of urine output data to a separate computer located at, for example, a nursing station 14, such that the data can be monitored in real time and/or stored in the patient's electronic record. The load cell 16, the amplifier 18 and the signal-processing module 26 are all powered by a battery and power management system 29.

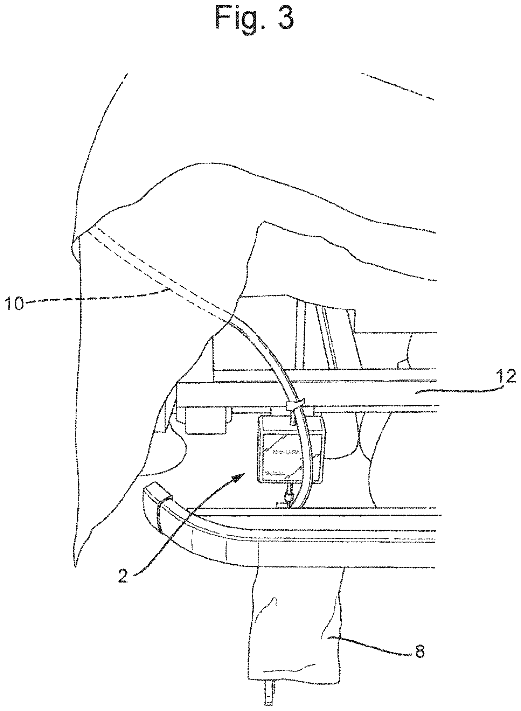

As shown in Figure 3, in the use, the gravimetric apparatus 2 is attached to the bed/trolley 12 of a catheterised patient (not shown). The catheter 10 is attached to the patient, and is fed directly into a urine collection bag 8, which is elevated off of the floor by the hook 4 of the in-line gravimetric apparatus 2. Thus, when the patient urinates, their urine flows into the urine collection bag 8 via the catheter 10. The gravimetric device 2 measures both the total amount of urine output, based on the weight of the urine collection bag 8, and also the rate of urine output, based on the change in weight of the collection bag 8 over time.

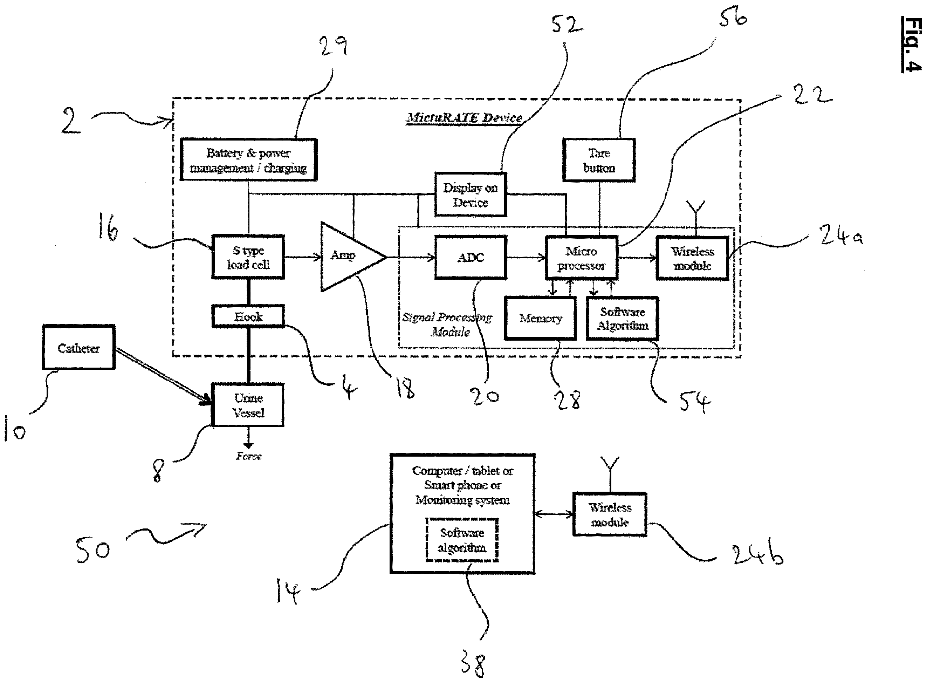

Figure 4 shows an embodiment of a urine detection system 50 including the gravimetric apparatus 2 described above. Data output of the microprocessor 22 is processed by software 54, and then transmitted directly (i.e. via a wired connection) to a display and/or input apparatus 52. Data (i.e. urine volume and output rate) is displayed in real time. The apparatus 2 includes a Tare button 56 to allow correction for the urine vessel 8 weight at the start of the measurement, when the vessel is emptied or when a sample is removed. If the alert signal notifies a "full vessel" status, the algorithm 54, in conjunction with the 'Tare' function 56, will recognise a replaced or emptied vessel 8 and append subsequent data to that already collected.

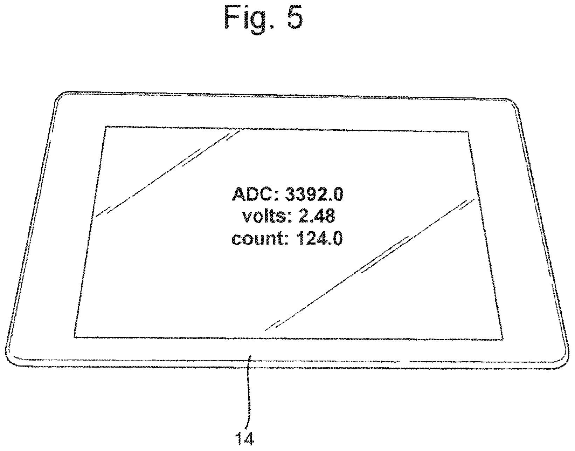

In addition, or alternatively, the processed data from the microprocessor 22 is fed to a wireless or wifi module 24a. The data emitted by the wireless/wifi module 24a is detected by a corresponding module 24b operably linked to a separate display 14 of a computer, mobile phone, smart phone, tablet or similar monitoring device, an example of which is shown in Figure 5, which may be located at a nursing station. The data is then converted by software 38 loaded on the computer/tablet/monitoring system 14 into a format that can be understood by a nurse, displayed on the screen and saved directly to the patient's electronic file. Data (i.e. urine volume and output rate) is displayed in real time.

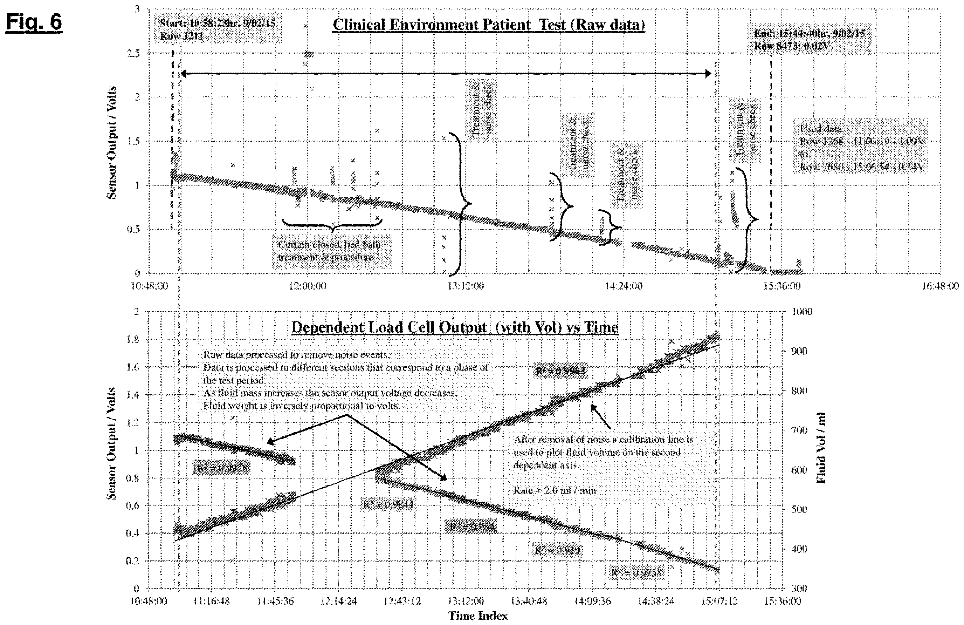

The upper panel of Figure 6 displays raw data collected from a catheterised patient using the apparatus 2 according to the invention. Raw data includes multiple digitised load cell values with a date-time stamp. The x-axis corresponds to time and the y-axis corresponds to sensor output, in Volts. The raw data of the upper panel of Figure 6 has been processed using MS Excel to remove "noise events" mentioned in the upper panel, such as "treatment and nurse check".

The data output of the lower panel of Figure 6 is the output, i.e. the descending line, of the urine output monitor. This data is inversely proportional to the total volume of urine output and is therefore used to calculate the total volume of urine output, i.e. the ascending line, of the catheterised patient. There is no medical data available from the subject; known as "Patient X" except that they were heavily sedated, had no renal complications and their fluid input was being managed at 180ml/hr (typical output is therefore expected to be between 1ml and 2ml/hr/kg). As expected for patient X, the urine output was steady. This was recorded by the apparatus 2 (see Figures 1-3) and included the output volume (at any point in time), average rate and output characteristics. The resolution of the continuous monitoring achieved by the apparatus 2 is such that any change in output quickly identifies oliguria, polyuria and anuria (or potential catheter blockage) in the patient.

### Example 2 - Applications of the gravimetric urine output measuring apparatus.

The apparatus 2 measures the urine output and flow rate of a catheterised patient in either the clinical or care home setting. The apparatus 2 works with any currently used collection bags 8 because it adopts an in-line measuring technique. This feature also mitigates any infection risk as there are no expensive sensors or dead volumes in the actual catheter tube 10 (difficult to sterilise micro volumes) or adaptors connected to existing catheter tubes. In fact, there is no change in the current "hung bag" configuration, except that the apparatus 2 is in-line with the bag 8; this makes the system very familiar with all medical and care staff. Unlike the existing technique of reading levels from a flexible bag (or rigid flow rate container), the apparatus 2 is consistently accurate and sensitive to small changes in collected fluid. Signal processing and algorithms are used to remove spurious data caused by routine intervention (a bed bath for example) and movement of the patient. During initial set-up by nursing staff the apparatus 2 is initiated (tare function for example) and subsequent changes (bag 8 emptied or urine sample removed) recorded by user interface on the apparatus 2 or automatically by the software algorithm 54.

The apparatus 2 can also include alarms for blockage, sudden change in output and/or full bag status. The apparatus 2 requires no leads to connect to a computer base station 14, which is achieved via a wireless link and has no external units, like a power supply or separate display (except data displayed wirelessly on a nursing station computer 14). There are two basic operating modes for the apparatus 2; "wireless monitoring mode", in which the raw data from the apparatus 2 is transmitted wirelessly to a nursing computer base station, tablet or laptop or smart phone 14, and "standalone operation mode", in which the apparatus 2 is completely independent and operated via a user interface on the surface of the apparatus 2.

Standalone operation lends itself to small scale use or facilities that do not have a well-developed IT infrastructure (possibly emerging countries). Nursing staff would still record the data manually, from the apparatus display, on common fluid balance charts. Supporting both modes of operation is the "display on device" feature that shows current values, recent fluid trends and alerts. Furthermore, this feature enables easy reading of the data at night by nursing staff without switching on the lights, a common complaint by patients.

When employed in a wireless monitoring mode the software installed on a separate computer 14 continuously monitors the urine output, providing a high resolution trend and history, which provides integrity of data and avoids possible falsification of data or incorrect entry of data by staff. The "forgotten catheter" is a common problem across the medical industry and the apparatus 2 offers a partial solution to this concern if it is used routinely for all catheterised patients by flagging use of apparatus 2 and by assumption of use of a catheter 10 via an electronic record. The apparatus 2 is also rugged being mainly solid state and hence can be used in a variety of environments e.g. hospital, care homes etc. The apparatus 2 is reusable as it can be moved and recharged for use easily. Finally, as there is no direct interface with the catheter 10 and due to its "sealed type box" design can be easily cleaned with wipes etc. to meet medical cleanliness requirements avoiding need for specialised cleaning protocols and facilities.

### References

[1] Mehta, R.L., ACUTE KIDNEY INJURY Urine output in AKI-the canary in the coal mine? Nature Reviews Nephrology, 2013. 9(10): p. 568-570.
[2] Hersch, M., S. Einav, and G. Izbicki, Accuracy and ease of use of a novel electronic urine output monitoring device compared with standard manual urinometer in the intensive care unit. Journal of Critical Care, 2009. 24(4).
[3] Kerr, M., et al., The economic impact of acute kidney injury in England. Nephrology Dialysis Transplantation, 2014. 29(7): p. 1362-1368.
[4] Ralib, A.M., et al., The urine output definition of acute kidney injury is too liberal. Critical Care, 2013. 17(3).
[5] Carter, C., Physiological observations and Early Warning Scoring tools within the deployed field hospital. Journal of the Royal Army Medical Corps, 2013. 159(4): p. 283-286.
[6] Bellomo, R., et al., Acute renal failure - definition, outcome measures, animal models, fluid therapy and information technology needs: the Second International Consensus Conference of the Acute Dialysis Quality Initiative (ADQI) Group. Critical Care, 2004. 8(4): p. R204-R212.
[7] Warren, J.W., Catheter-associated urinary tract infections. Infectious Disease Clinics of North America, 1997. 11(3): p. 609.

## Claims

1. A urine weighing apparatus (2) comprising support means (4) configured to support a urine collection vessel (8), a transducer (16) for converting the weight of the vessel into an electrical signal, and a processor (22) for processing the electrical signal to continuously calculate the weight of the vessel, and thereby calculate the weight of urine therein and allow the urine output rate to be determined, wherein the transducer is a load cell and wherein the load cell is an S-type load cell with a minimum of two strain gauges configured to measure both positive and negative changes in force,
wherein the apparatus is configured to process the data to remove unwanted changes and data artefacts due to shock inputs, clinical interventions and/or movement of the apparatus and/or subject,
wherein the processor (22) is programmed with an algorithm which manages the fluid volume data, including interventions, which abruptly change the vessel's weight, and
wherein if an alert signal produced by the apparatus notifies a "full vessel" status, the algorithm, in conjunction with a 'Tare' function of the apparatus, recognizes a replaced or emptied vessel, and appends subsequent data to that already collected.

2. An apparatus according to claim 1, wherein the apparatus is configured to measure the weight of the vessel, and thereby calculate, in real-time, the volume of urine therein.

3. An apparatus according to any one of the preceding claims, wherein the apparatus is configured to calculate voided urine volume at a plurality of time points, and then calculate a rate of change of urine volume over the plurality of time points, and/or wherein the support means (4) is a hook, a latched hook, a clip or cradle which supports the collection vessel.

4. An apparatus according to any one of the preceding claims, wherein the urine collection vessel (8) is a urine collection bag, and/or wherein the apparatus (2) comprises an amplifier (18) configured to amplify the electronic analogue signal generated by the transducer (16).

5. An apparatus according to any one of the preceding claims, wherein the apparatus comprises a wireless module (24a) for transmission of data to a separate electronic device, computer, server (web page), laptop, tablet or smart phone.

6. An apparatus according to any one of the preceding claims, wherein the apparatus is capable of detecting a change in voided urine volume of less than 1 ml, 0.75 ml, 0.5 ml, 0.3 ml or 0.1 ml.

7. An apparatus according to any one of the preceding claims, wherein the device further comprises a power supply (29), wherein the power supply (29) uses inductive charging.

8. Use of the apparatus according to any one of claims 1 to 7, for weighing urine.

9. A method of measuring the urine output from a subject, the method comprising measuring urine output from a subject to which the apparatus according to any one of claims 1 to 7 is attached.

10. The apparatus (2) according to any one of claims 1 to 7, further comprising a urine collection vessel (8).

11. The device according to claim 10, wherein the device further comprises a delivery means (10) for delivering urine from a subject to the urine collection vessel.

12. The apparatus according to claim 11, wherein the delivery means (10) is a catheter.

13. The apparatus according to any one of claims 1 to 7, further comprising at least one device (14) for receiving data transmitted by the apparatus, which corresponds to the weight of the urine output.

## Patentansprüche

1. Urinwägevorrichtung (2), umfassend Stützmittel (4), die konfiguriert sind, um einen Urinsammelbehälter (8) zu stützen, einen Wandler (16) zum Umwandeln des Gewichts des Behälters in ein elektrisches Signal und einen Prozessor (22) zum Verarbeiten des elektrischen Signals, um das Gewicht des Behälters kontinuierlich zu berechnen und dadurch das Gewicht von Urin darin zu berechnen und zu ermöglichen, dass die Urinausscheidungsrate bestimmt wird, wobei der Wandler eine Lastzelle ist und wobei die Lastzelle eine S-Typ-Lastzelle mit mindestens zwei Dehnungsmessstreifen ist, die konfiguriert sind, um sowohl positive als auch negative Kraftänderungen zu messen,
wobei die Vorrichtung konfiguriert ist, um die Daten zu verarbeiten, um unerwünschte Änderungen und Datenartefakte aufgrund von Stoßeinwirkungen, klinischen Eingriffen und/oder Bewegung der Vorrichtung und/oder des Subjekts zu entfernen,
wobei der Prozessor (22) mit einem Algorithmus programmiert ist, der die Flüssigkeitsvolumendaten verwaltet, beinhaltend Eingriffe, die das Gewicht des Behälters abrupt verändern, und
wobei, wenn ein Warnsignal, das durch die Vorrichtung produziert wird, einen Status "voller Behälter" anzeigt, der Algorithmus in Verbindung mit einer Tara-Funktion der Vorrichtung einen ersetzten oder geleerten Behälter erkennt und weitere Daten an die bereits gesammelten anfügt.

2. Vorrichtung nach Anspruch 1, wobei die Vorrichtung konfiguriert ist, um das Gewicht des Behälters zu messen und dadurch in Echtzeit das Volumen an Urin darin zu berechnen.

3. Vorrichtung nach einem der vorhergehenden Ansprüche, wobei die Vorrichtung konfiguriert ist, um geleertes Urinvolumen zu einer Vielzahl von Zeitpunkten zu berechnen und dann eine Änderungsrate an Urinvolumen über die Vielzahl von Zeitpunkten zu berechnen, und/oder wobei das Stützmittel (4) ein Haken, ein verriegelter Haken, eine Klammer oder eine Halterung ist, der/die den Sammelbehälter stützt.

4. Vorrichtung nach einem der vorhergehenden Ansprüche, wobei der Urinsammelbehälter (8) ein Urinsammelbeutel ist und/oder wobei die Vorrichtung (2) einen Verstärker (18) umfasst, der konfiguriert ist, um das elektronische Analogsignal, das durch den Wandler (16) erzeugt wird, zu verstärken.

5. Vorrichtung nach einem der vorhergehenden Ansprüche, wobei die Vorrichtung ein drahtloses Modul (24a) zur Übertragung von Daten an ein separates elektronisches Gerät, einen Computer, einen Server (Webseite), einen Laptop, ein Tablet oder ein Smartphone umfasst.

6. Vorrichtung nach einem der vorhergehenden Ansprüche, wobei die Vorrichtung in der Lage ist, eine Änderung an geleertem Urinvolumen von weniger als 1 ml, 0,75 ml, 0,5 ml, 0,3 ml oder 0,1 ml zu erfassen.

7. Vorrichtung nach einem der vorhergehenden Ansprüche, wobei das Gerät ferner eine Stromversorgung (29) umfasst, wobei die Stromversorgung (29) induktives Laden verwendet.

8. Verwendung der Vorrichtung nach einem der Ansprüche 1 bis 7 zum Wiegen von Urin.

9. Verfahren zum Messen der Urinausscheidung von einem Subjekt, wobei das Verfahren Messen von Urinausscheidung von einem Subjekt umfasst, an dem die Vorrichtung nach einem der Ansprüche 1 bis 7 angebracht ist.

10. Vorrichtung (2) nach einem der Ansprüche 1 bis 7, ferner umfassend einen Urinsammelbehälter (8).

11. Gerät nach Anspruch 10, wobei das Gerät ferner ein Abgabemittel (10) zum Abgeben von Urin von einem Subjekt an den Urinsammelbehälter umfasst.

12. Vorrichtung nach Anspruch 11, wobei das Abgabemittel (10) ein Katheter ist.

13. Vorrichtung nach einem der Ansprüche 1 bis 7, ferner umfassend zumindest ein Gerät (14) zum Empfangen von Daten, die durch die Vorrichtung übertragen werden, die dem Gewicht der Urinausscheidung entsprechen.

## Revendications

1. Appareil de pesage d'urine (2) comprenant des moyens support (4) conçus pour supporter un récipient de collecte d'urine (8), un transducteur (16) destiné à convertir le poids du récipient en un signal électrique, et un processeur (22) destiné à traiter le signal électrique afin de calculer en continu le poids du récipient, et ainsi calculer le poids de l'urine en son sein et permettre la détermination du taux de débit d'urine, ledit transducteur étant une cellule de charge et ladite cellule de charge étant une cellule de charge de type S avec un minimum de deux jauges de contrainte configurées pour mesurer à la fois les changements positifs et négatifs de force,
ledit appareil étant configuré pour traiter les données afin de supprimer les modifications indésirables et les artefacts de données dus aux entrées de choc, aux interventions cliniques et/ou au déplacement de l'appareil et/ou du sujet,
ledit processeur (22) étant programmé avec un algorithme qui gère les données de volume de fluide, y compris les interventions qui modifient brusquement le poids du récipient, et
si un signal d'alerte produit par l'appareil notifie un état de « récipient plein », ledit algorithme, en conjonction avec une fonction « Tare » de l'appareil, reconnaissant un récipient remplacé ou vidé, et ajoutant des données ultérieures à celles déjà collectées.

2. Appareil selon la revendication 1, ledit appareil étant configuré pour mesurer le poids du récipient et ainsi calculer, en temps réel, le volume d'urine en son sein.

3. Appareil selon l'une quelconque des revendications précédentes, ledit appareil étant configuré pour calculer le volume d'urine évacué à une pluralité de points temporels, puis calculer un taux de changement de volume d'urine sur la pluralité de points temporels, et/ou ledit moyen support (4) étant un crochet, un crochet verrouillé, une pince ou un socle qui supporte le récipient de collecte.

4. Appareil selon l'une quelconque des revendications précédentes, ledit récipient de collecte d'urine (8) étant une poche de collecte d'urine, et/ou ledit appareil (2) comprenant un amplificateur (18) configuré pour amplifier le signal analogique électronique généré par le transducteur (16).

5. Appareil selon l'une quelconque des revendications précédentes, ledit appareil comprenant un module sans fil (24a) destiné à la transmission de données à un dispositif électronique distinct, un ordinateur, un serveur (page Web), un ordinateur portable, une tablette ou un téléphone intelligent.

6. Appareil selon l'une quelconque des revendications précédentes, ledit appareil étant capable de détecter un changement dans le volume d'urine évacué inférieur à 1 ml, 0,75 ml, 0,5 ml, 0,3 ml ou 0,1 ml.

7. Appareil selon l'une quelconque des revendications précédentes, ledit dispositif comprenant en outre une alimentation électrique (29), ladite alimentation électrique (29) utilisant une charge inductive.

8. Utilisation de l'appareil selon l'une quelconque des revendications 1 à 7, pour le pesage d'urine.

9. Méthode de mesure du débit d'urine d'un sujet, le procédé comprenant la mesure du débit d'urine d'un sujet auquel l'appareil selon l'une quelconque des revendications 1 à 7 est fixé.

10. Appareil (2) selon l'une quelconque des revendications 1 à 7, comprenant en outre un récipient de collecte d'urine (8).

11. Dispositif selon la revendication 10, ledit dispositif comprenant en outre un moyen de distribution (10) destiné à distribuer l'urine d'un sujet au récipient de collecte d'urine.

12. Appareil selon la revendication 11, ledit moyen de distribution (10) étant un cathéter.

13. Appareil selon l'une quelconque des revendications 1 à 7, comprenant en outre au moins un dispositif (14) destiné à la réception de données transmises par l'appareil, qui correspondent au poids du débit d'urine.
